# EUROPEAN PATENT SPECIFICATION

(11) **EP 0 755 212 B1**
(45) Date of publication and mention of the grant of the patent: **01.03.2000**
(21) Application number: 95917316.2
(22) Date of filing: 12.04.1995
(51) Int. Cl.: A47K 10/16, B31F 1/07, D21H 27/40

(54) **A CELLULOSE CLOTH FOR HYGIENE**
ZELLSTOFF-HYGIENETUCH
ETOFFE CELLULOSIQUE DESTINEE A L'HYGIENE

(30) Priority: 12.04.1994 DE 9406026 U
(43) Date of publication of application: 29.01.1997
(73) Proprietor: THE PROCTER & GAMBLE COMPANY, Cincinnati, Ohio 45202 (US)
(72) Inventor: REINHEIMER, Horst, 90562 Heroldsberg (DE); HILBIG, Klaus, 90522 Oberasbach (DE); SCHMITT, Werner, 90562 Heroldsberg (DE)
(74) Representative: Hübner, Gerd, Dipl.-Phys.
(86) International application number: EP9501372
(87) International publication number: WO9527429

(56) References cited:
- EP-A- 0 265 298
- EP-A- 0 436 170
- WO-A-92/14605
- WO-A-93/11929
- NL-A- 8 400 146
- US-A- 3 414 459

## Description

The invention relates to a process of embossing spots into a cellulose cloth for hygiene with the features specified in the preamble of claim 1.

Cellulose cloths for hygiene which are known and normal, for example, as handkerchiefs, cosmetic cloths, household cloths or the like generally consist of several layers of tissue which are connected to one another by a marginal embossed pattern. Embossed patterns of this type consist of a plurality of individual spot-shaped impressions which deform the layers of tissue transversely to the direction of the layers.

These impressions are usually introduced from one side of the cellulose cloth using an embossing roller which operates against a smooth, optionally flexible, opposing roller. This mode of operation is known as random embossing. It is also possible to work with a pair of steel rollers consisting of a male-moulding roller and a female-moulding roller by the so-called union embossing process. With random embossing and also with union embossing, the impressions are curved concavely inwardly on one side of the cellulose cloth, but are correspondingly convexly raised on the other side of the cloth.

The above-described known embossing technique has various drawbacks. For example, the cloth receives a rough nature in the region of the raised impressions which considerably impairs the typical requirements for use of cellulose cloths for hygiene such as softness and skin-friendliness. To minimise these drawbacks, the impressions are limited to a narrow marginal region in known cellulose cloths for hygiene. This restricts, on the one hand, the durability of the connection between the individual layers of tissue because there are to be no embossments in the large-area central region of the cloth. On the other hand, the freedom of design of the embossed pattern which serves as a design element in addition to its connecting function is also considerably restricted.

Furthermore, the one-sided embossment of known cellulose cloths projects markedly from the back of the cloth with the result that a folded cloth with such embossments requires more space.

US-A-3,414,459 discloses a compressible laminated paper structure, which is produced by a first step of embossing single layers of tissue followed by a separate and distinct step of connecting two or more tissue layers together. In fact, this reference teaches to emboss patents of discrete protuberances on two separate sheets by the action of nips formed by steel embossing rolls, which counteract with the rubber-covered rolls. Thus, US-A-3,414,459 discloses a typical random embossing process of single sheets, which are connected afterwards.

The object of the invention is to provide a process of embossing spots into a cellulose cloth for hygiene, which leads to markedly improved embossments.

This object is achieved by the features specified in the characterizing part of claim 1, according to which the process of the invention is characterized by the step of guiding multi-layered unembossed cellulose cloths between the pair of embossing rollers so that the layers of unembossed cellulose cloth are connected to one another by the embossing action. This process leads to a cellulose cloth, in which the impressions are no longer applied on one side but on both sides and are formed from embossed spots which issue from the two outer layers of tissue and are curved concavely inwardly in each case. These embossed spots are mutually aligned transversely to the layer direction, in other words rest head to head with their internal base regions with the result that the layers of tissue are connected to one another there by the embossing pressure.

Owing to this design of the impressions, the cellulose cloth does not have raised embossed regions projecting beyond the outer layers of tissue. The roughening effect mentioned at the outset is therefore avoided. This means that the cloth is particularly soft and skin-friendly. Furthermore, owing to this design, the impressions can be provided in any design not only in the marginal region but virtually over the entire area of the cellulose cloth. The embossed pattern therefore represents a freely creatable design element. An embossed connection of the layers of tissue not restricted to the marginal region can also be achieved so the layered assembly of the cloth is considerably improved in comparison with the prior art. A further advantage of the cellulose cloths produced according to the invention is that the impressions no longer project, so the space required by a stack of folded cellulose cloths is no longer dependent on the embossment but only on the smoothness of the cloths - that is on the thickness of the layers of tissue achieved by appropriate calendering. This means that, in practice, the volume of a conventional pack of ten cellulose cloths for hygiene can be reduced by about 20% to 50%.

Further features, details and advantages of the invention can be inferred from the sub-claims and the following description in which an embodiment of the subject of the invention is described in detail with reference to the accompanying drawings.

Figure 1 shows partial plan views of cellulose cloths for hygiene with different embossed patterns.

Figure 2 is a greatly enlarged schematic section transversely to the direction of layers through a cellulose cloth for hygiene according to the prior art and a cloth produced according to the invention.

Figure 3 is a schematic section through an embossing tool for a cellulose cloth for hygiene.

Figure 4 is a schematic perspective view of embossing mounds on the embossing tool.

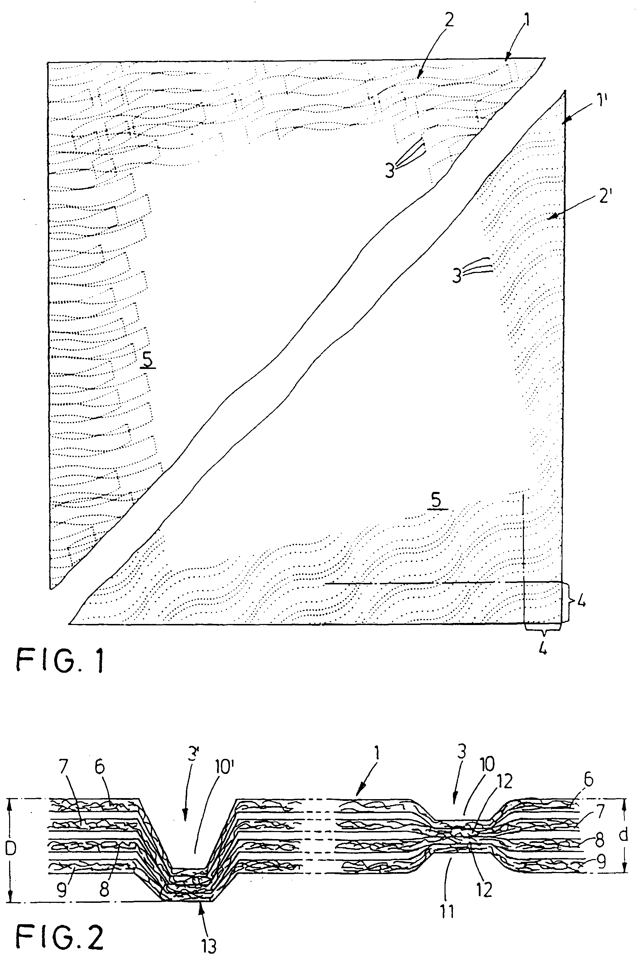

Figure 1 shows details of two cellulose cloths 1 for hygiene of the type used, for example, as disposable handkerchiefs. The two cloths shown have different impressions 2, 2' which are formed from a plurality of individual spot-shaped impressions 3 in both cases. The impressions 3 in the embossed pattern 2 of the cellulose cloth for hygiene 1 shown in the top left-hand corner form curved over-lapping bars. The embossed pattern 2' in the cellulose cloth 1' shown on the bottom right is formed by impressions 3 arranged in undulating lines. Both embossed patterns 2, 2' extend beyond a narrow marginal region 4 of the cloth 1, 1' into the central region 5 thereof, but the embossed patterns 2, 2' can also cover the entire area of the cellulose cloths 1, 1', not shown. The marginal region 4 determines the width to which the formerly used marginal embossed patterns are usually restricted in cellulose cloths according to the prior art.

Figure 2 serves as further illustration of the distinction between a cellulose cloth produced according to the invention (shown in the right-hand part of Figure 2) and the prior art (shown in the left-hand part of Figure 2). The cellulose cloth 1 basically consists, for example, of four individual layers of tissue 6, 7, 8, 9 with a weight per unit area of about 15 g/m² in each case. The layers 6 to 9 are connected to one another by the impressions 3 with which the layers of tissue 6 to 9 are deformed transversely to the direction of the layers and are attached to one another by the embossing pressure.

As shown in the right-hand part of Figure 2, the impressions 3 in the cellulose cloth 1 produced according to the invention are formed in each case by two embossed spots 10, 11 which are mutually aligned transversely to the direction of the layers and curve concavely inwardly from the two outer layers of tissue 6, 9 in each case. The inner layers of tissue 7, 8 are thus enclosed. The embossed spots 10, 11 are introduced in such a way that the layers of tissue 6 to 9 are squeezed together by the embossing action in the base region 12 of the embossed spots 10, 11 and are therefore permanently connected.

The impressions 3' according to the prior art are shown in the left-hand part of Figure 2. In this case, an embossed spot 10' is introduced into the cloth from one side of the cellulose cloth, for example originating from the tissue layer 6. The cloth therefore has a convex bulge 13 on the remote side.

As demonstrated by a comparison between the left-hand and right-hand sides of Figure 2, the cellulose cloth 1 produced according to the invention with the embossed spots 10, 11 on both sides remains smooth on both sides as it does not have any bulges. In this respect, the cellulose cloth does not have a different front and back either, as is the case with the cloth according to the prior art. Furthermore, the effective thickness of the cloth is not increased by the embossed spots 10, 11, as in the prior art. As shown in the left-hand part of Figure 2, the cloth according to the prior art has an effective thickness D which is substantially increased by the bulges 13 of the embossed spots 10'. The thickness d of the cellulose cloth produced according to the invention, on the other hand, depends only on the smoothness of the individual layers of tissue 6 to 9.

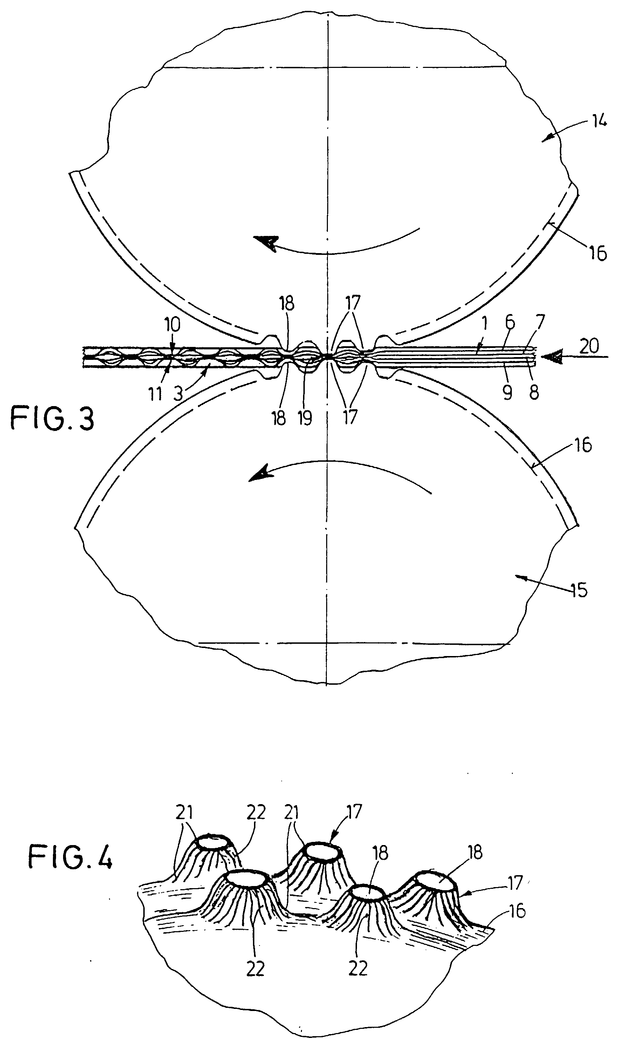

Figures 3 and 4 illustrate the embossing process for introducing the embossed spots 10, 11 into the cellulose cloth 1. A pair of embossing rollers 14, 15, which have embossing mounds 17 arranged congruently according to the respective embossed pattern on their external surface 16 in each case, is used as embossing tool. The embossing rollers 14, 15 are arranged and controlled in such a way that the embossed mounds 17 are arranged exactly head-to-head in each case but do not touch one another. Between the faces 18 of the embossing mounds 17, a distance between the faces 18 of fractions of a millimetre remains in the embossing gap 19 through which the multi-layered unembossed cellulose cloth 1 is guided. This distance is adjusted in such a way that the tissue layers 6 to 9 are squeezed together as they pass through the embossing gap 19. Furthermore, the two embossing rollers 14, 15 are produced from refined steel and are therefore inherently rigid. With an appropriately precise arrangement and control of the embossing rollers 14, 15, an embossed result is achieved which is more reproducible than in the prior art where a profiled embossing roller and a smooth flexible opposing roller into which the embossing mounds of the embossing roller are impressed are used.

As shown in Figure 4, the individual embossing mounds 17 are substantially truncated cone shaped, the transitions 21 from the external surface 16 or face 18 into the flanks 22 of the embossing mounds being smooth-surfaced and rounded. An embossing process which preserves the material is thus achieved.

## Claims

1. A process of embossing spots (10, 11) into a cellulose cloth (1), by guiding the cellulose cloth (1) between a pair of embossing rollers (14, 15) which have embossing mounds (17) arranged congruently according to respective embossed patterns on their external surface (16), the embossing mounds (17) being arranged exactly head-to-head in each case and being spaced apart to provide an embossing gap (19), the process being characterised by the step of guiding multi-layered unembossed cellulose cloth (6, 7, 8, 9) between the pair of embossing rollers (14, 15) so that the layers of unembossed cellulose cloth (6, 7, 8, 9) are connected to one another by the embossing action.

2. A process according to claim 1 wherein the embossing rollers (14, 15) are inherently rigid.

3. A process according to claim 2 wherein the embossing rollers (14, 15) are made from refined steel.

## Patentansprüche

1. Verfahren zum Einprägen von Punkten (10, 11) in ein Zellstofftuch (1) durch Führen des Zellstofftuchs (1) zwischen ein Paar von Prägewalzen (14, 15), die Prägenoppen (17) aufweisen, die gemäß dem jeweiligen Prägemuster an ihren Außen-Oberflächen (16) kongruent angeordnet sind, wobei die Prägenoppen (17) jeweils exakt Kopf-an-Kopf angeordnet und voneinander beabstandet sind, um einen Prägespalt (19) zu bilden, wobei das Verfahren gekennzeichnet ist durch den Schritt, daß ein mehrlagiges, nicht-geprägtes Zellstofftuch (6, 7, 8, 9) zwischen dem Paar von Prägewalzen (14, 15) hindurchgeführt wird, so daß die Lagen des nicht-geprägten Zellstofftuchs (6, 7, 8, 9) miteinander durch die Prägewirkung verbunden werden.

2. Verfahren nach Anspruch 1, wobei die Prägewalzen (14, 15) in sich starr sind.

3. Verfahren nach Anspruch 2, wobei die Prägewalzen (14, 15) aus Edelstahl hergestellt sind.

## Revendications

1. Procédé de gaufrage de parties (10, 11) dans une étoffe de cellulose (1), par guidage de l'étoffe de cellulose (1) entre une paire de rouleaux de gaufrage (14, 15) qui ont des bosses de gaufrage (17) agencées de manière congruente selon des motifs gaufrés respectifs sur leur surface externe (16), les bosses de gaufrage (17) étant agencées exactement tête-à-tête dans chaque cas et étant espacées afin de fournir un intervalle de gaufrage (19), le procédé étant caractérisé par l'étape de guidage de l'étoffe de cellulose non gaufrée (6, 7, 8, 9) multicouche entre la paire de rouleaux de gaufrage (14, 15), afin que les couches de l'étoffe de cellulose non gaufrée (6,7, 8, 9) soient reliées les unes aux autres sous l'effet du gaufrage.

2. Procédé selon la revendication 1, dans lequel les rouleaux de gaufrage (14, 15) sont rigides de manière inhérente.

3. Procédé selon la revendication 2, dans lequel les rouleaux de gaufrage (14, 15) sont réalisés en acier fin.
